# EUROPÄISCHE PATENTSCHRIFT

(11) **EP 1 219 322 B1**
(45) Veröffentlichungstag und Bekanntmachung des Hinweises auf die Patenterteilung: **16.03.2005**
(21) Anmeldenummer: 01250414.8
(22) Anmeldetag: 26.11.2001
(51) Int. Cl.: A61N 5/06

(54) **Therapeutische Bestrahlungsanordnung**
Therapeutical irradiation arrangement
Ensemble d'irradiation thérapeutique

(30) Priorität: 02.01.2001 DE 10100662
(43) Veröffentlichungstag der Anmeldung: 03.07.2002
(73) Patentinhaber: OptoMed Optomedical Systems GmbH, 12489 Berlin (DE)
(72) Erfinder: Wilkens, Jan H., Dr. med, 66242 Hamburg (DE)
(74) Vertreter: Effert, Bressel und Kollegen

(56) Entgegenhaltungen:
- WO-A-00/02491
- DE-A- 19 838 304
- DE-A- 19 852 524

## Beschreibung

Die Erfindung betrifft eine therapeutische Bestrahlungsanordnung, insbesondere zur chronischen Behandlung von ganz oder teilweise zellvermittelten Entzündungen der Haut und der inneren Organe, viralen und anderen infektiösen Erkrankungen wie beispielsweise Prioneninfektionen.

Primär T-Zell-vermittelte Hauterkrankungen, wie beispielsweise atopische Dermatitis (Neurodermitis), cutanes T-Zell-Lymphom, Lichen ruber und Psoriasis beruhen auf einem Hautinfiltrat von aktivierten T-Lymphozyten des eigenen Körpers. Insbesondere von Neurodermitis sind verstärkt immer mehr Neugeborene und Kinder betroffen. Aufgrund der entzündeten Hautpartien, sowie des damit verbundenen Juckreizes ist diese Erkrankung sowohl physiologisch als auch psychologisch eine schwere Belastung.

Die bisher bekannten Therapien zur Behandlung von Neurodermitis lassen sich im wesentlichen in zwei Klassen unterteilen, nämlich die Chemotherapie und die UVA1-Lichttherapie.

Bei der Chemotherapie ist der derzeitige Goldstandard in der Behandlung der atopischen Dermatitis die Glukokortikaidtherapie. Bei dieser Therapie kommt es sowohl nach systemischer als auch nach topischer Anwendung zu zum Teil schwerwiegenden Nebenwirkungen. Alternative Verfahren zur Behandlung der Neurodermitis beinhalten die Therapie mit stark immunmodulierenden Pharmaka, wie beispielsweise FK 506 oder Cyclosporin A, über deren Langzeitfolgen noch keine Erfahrungen vorliegen.

Die UVA 1- Lichttherapie hat sich als effektiv zur Behandlung von akuten Neurodermitisschüben, der Urticaria pigmentosa und lokalisierten Sklerodermie erwiesen. Zur Zeit werden für die UVA 1 - Therapie nach Meffert und die UVA 1 - Therapie nach Krutmann zwei Gerätetypen angeboten. Die UVA 1 -Therapie nach Meffert arbeitet breitbandig zwischen 340 und 500 nm, die UVA- Therapie nach Krutmann bei 340 - 400 nm.

Einen sehr guten Überblick über den Stand der Technik in der UVA 1 -Therapie bietet "Stellung zur Qualitätssicherung in der UVA 1-Phototherapie, Fassung der Untergruppe Foto-(Chemo)Therapie und -Diagnostik der Subkommission physikalische Verfahren in der Dermatologie, Mai 1998", sowie die "Richtlinien zur Qualitätssicherung in der Foto-(Chemo)Therapie und Diagnostik", die in "Krutmann, S., Hönigsmann, H.: Handbuch der Dermatologischen Phototherapie und -Diagnostik, Springer-Verlag, Heidelberg, pp. 392 -395" veröffentlicht ist. Als Langzeitrisiken sind dort eine vorzeitige Hautalterung und Karzinogenität aufgeführt. Aufgrund dieser Sachlage ist dort explizit ausgeführt, daß eine Anwendung von mittleren und hohen Dosen von UVA1 im Kindesalter nicht zu empfehlen sind. Damit ist jedoch gerade die größte betroffene Gruppe von Neurodermitis ausgenommen.

Bei einer Bestrahlung kommt es zu zwei Typen von DNS-Schäden, nämlich der Erzeugung von Pyrimidindimeren und oxidativen DNS-Modifikationen. Die erste Gruppe der DNS-Schäden ist über körpereigene Reparaturenzyme vergleichsweise schwer zu reparieren und die Induktion derartiger Schäden ist karzinogen. Das Maximum dieser Schäden wird durch Bestrahlung um 290 nm ausgelöst, wobei bei 400 nm der Grad der Schädigung um den Faktor 10.000 geringer ist und bei Wellenlängen über 425 nm nicht mehr nachweisbar ist. Diese Art der Schädigungen wird auch als CPD (Cyclobuthanpyrimidinphotodimere) bezeichnet.

Hiervon unterscheidet sich der Typ der sogenannten indirekten DNS-Schädigung der vermutlich über die Photonenanregung zellulärer Chromophore bewirkt wird. Diese Chromophoren erzeugen reaktive Sauerstoffspezies wie beispielsweise Singulettsauerstoff. Der hierdurch ausgelöste DNS-Schaden (8-Hydroxyguanin) wird durch die sogenannte FPG-Protein-Endonuklease repariert. Es handelt sich hierbei um vergleichsweise leichte Schäden im Bereich der DNS-Quervernetzung und nicht um DNS-Strangbrüche oder Basenverlust. Dies wurde durch Versuche mit transgenen Mäusen ohne FPG-Reparaturenzyme belegt, die dennoch keine erhöhte Tumorrate aufwiesen.

Aus der DE 198 52 524 A1 ist eine Bestrahlungseinrichtung für therapeutischen und kosmetische Zwecke bekannt, umfassend mindestens eine optische Strahlungsquelle, die auf einer zu bestahlenden Fläche im Wellenlängenintervall von 400 - 440 nm eine Bestrahlungsstärke von mindestens 20 mW/cm² erzeugt und im Wellenlängenintervalle von 340 - 400 nm eine Bestrahlungsstärke von weniger als 2 mW/cm² erzeugt, wobei die Bestrahlungseinrichtung von Primär T-Zell-vermittelten Hauterkrankungen, insbesondere atopischer Dermatitis (Neurodermitis), cutanen T-Zell-Lymphom, Lichen ruber, Alopecia areata, systemischen Lupus erythematodes und Psoriasis verwendet wird. Durch die überraschende Wirksamkeit der Strahlung im Bereich von 400-440 nm auf die T-Zellen ist es somit möglich, eine Bestrahlungseinrichtung zur Behandlung von T-Zell-vermittelten Hauterkrankungen zu schaffen, die einerseits bisher kaum behandelbare Hauterkrankungen wie Lichen ruber zu behandeln ermöglicht und andererseits aufgrund der um Zehnerpotenzen geringeren Karzinogenität gegenüber UVA auch eine Behandlung von Kindern ermöglicht. Allerdings stellt sich bei dieser Bestrahlungsanordnung das Problem, ob bei Langzeittherapien von chronischen Erkrankungen trotz der 10.000-fach geringeren Karziogenität nicht doch CPD-Schäden auftreten können.

Der Erfindung liegt daher das technische Problem zugrunde, eine therapeutische Bestrahlungsanordnung, insbesondere zur chemischen Behandlung von ganz oder teilweise zellvermittelten Entzündungen der Haut und der inneren Organe, viralen und anderen infektiösen Erkrankungen, wie beispielsweise Prioneninfektionen, zu schaffen, die potentielle DNS-Schädigungen wie CPD-Schäden auch bei Langzeittherapien weitgehend vermeidet.

Die Lösung des technischen Problems ergibt sich durch den Gegenstand des Patentanspruchs 1. Weitere vorteilhafte Ausgestaltungen der Erfindung ergeben sich aus den Unteransprüchen.

Hierzu erzeugt die breitbandige optische Bestrahlungsquelle im Wellenlängenbereich von 430 - 500 nm eine Bestrahlungsstärke von mindestens 60 mW/cm² und im Wellenlängenbereich von 340 - 430 nm eine Bestrahlungsstärke von weniger als 20 mW/cm². Die Angaben der Bestrahlungsstärke beziehen sich dabei auf die Oberfläche eines Patienten. Dabei wird die Erkenntnis ausgenutzt, daß im angegebenen Wellenlängenbereich ein phototherapeutisches Fenster existiert, dessen Mechanismus über Flavine bzw. Flavinproteine vermittelt wird, die im angegebenen Wellenlängenintervall Singulettsauerstoff erzeugen. Durch die Bestrahlungsstärke von größer 60 mW/cm² wird dabei eine therapeutisch wirksame Menge an Singulettsauerstoff erzeugt, so daß die in der DE 198 52 524 A1 erwähnten therapeutischen Wirkungen eintreten, wobei aufgrund der verwendeten Wellenlängen oberhalb von 425 nm jegliche DNS-Schädigungen wie CPD auch bei Langzeittherapien auszuschließen sind. Ein weiterer Vorteil ist, daß mittels der Bestrahlungsanordnung eine Prioneninaktivierung möglich ist. Prionen sind vollkommen inert gegen ionisierende Strahlung, UVC-Strahlung oder Hitze bis zu 140°C. Die membranbeständige Anlagerung dieser infektiösen Eiweißpartikel an Lymphozytenmembranen macht diese dagegen empfindlich für photochemische oxidative Mechanismen, insbesondere für Singulettsauerstoff. Aufgrund der bisherigen Erkenntnisse scheint die Bestrahlungsanordnung darüber hinaus prinzipiell zur Inaktivierung von HIV-Viren geeignet zu sein. Bisher ist es bereits bekannt, daß mit Hilfe des Benzoporphyrins BPD und einer Lichtbehandlung sowohl das vesikuläre Stromatitis-Virus als auch das felline Leukämievirus behandelt werden konnten. Auch das HIV-Virus, selbst in der HCT-resistenten Form, führte zu einer Nicht-Infektiösität von infiziösem Material, wobei Singulett-Sauerstoff für die irreversible Schädigung der Virushülle verantwortlich gemacht wird. Die proliferierenden Zellen, die aktiv HIV-Viren replizieren, werden daher bevorzugt vernichtet, während latent infizierte Zellen der Photodestruktion entkommen. Es ist somit möglich, den höheren Porphyrinspiegel in den aktiv replizierenden Zellen für deren Ausschaltung auszunutzen. Vorzugsweise wird die Bestrahlungsstärke im Bereich von 340 - 430 nm kleiner 10 mW/cm² weiter vorzugsweise kleiner 2 mW/cm² eingestellt.

In einer bevorzugten Ausführungsform wird die Bestrahlungsstärke von größer 60 mW/cm² im Wellenlängenintervall von 440 - 480 nm erzeugt, da die Absorptionsmaxima bei 450 bzw. 470 nm von 3-Methyl-Lumiflavin liegen, so daß die Ausbeute an Singulettsauerstoff bei gleicher Bestrahlungsstärke erhöht wird.

In einer weiteren bevorzugten Ausführungsform wird eine Bestrahlungsstärke von größer 100 mW/cm², vorzugsweise größer 200 mW/cm² im relevanten Wellenlängenintervall erzeugt. Neben einer Verkürzung der Bestrahlungsdauer zur Verabreichung einer Dosis von beispielsweise 200 J/cm² ist der besondere Vorteil, daß sicher patientenindividuelle Schwellwerte überschritten werden. So hat sich in klinischen Untersuchungen herausgestellt, daß einzelne Patienten erst bei sehr großen Bestrahlungstärken ab 100 mW/cm² ansprechen, wobei die genauen Ursachen für diese Schwellwerte noch nicht genau spezifizierbar sind.

In einer weiteren bevorzugten Ausführungsform umfaßt die Bestrahlungsanordnung eine Einrichtung zur inspiratorischen und/oder topischen Sauerstoffzugabe. Dadurch wird der Sauerstoffgehalt in den zu behandelnden Körperpartien erhöht, was wiederum die Bildung von reaktiven O₂-Spezies verbessert.

Die Einrichtung zur inspiratorischen Sauerstoffzugabe kann beispielsweise als Sauerstoffflasche mit angeschlossener Nasen-Mund-Maske ausgebildet sein. Vorzugsweise umfaßt die Einrichtung jedoch einen gasdichten Hüllkörper, in dem eine reine Sauerstoffatmosphäre erzeugbar ist. Insbesondere bei lokalen oberflächlichen Entzündungen kann der Hüllkörper als Glocke ausgebildet sein, der dann lokal auf die zu behandelnde Stelle aufgesetzt und mit Sauerstoff geflutet wird, so daß dann über die Haut eine topische Sauerstoffzufuhr stattfindet. Besonders vorteilhaft ist jedoch ein Gehäuse, in dem der Patient vollständig liegt oder steht. Anschließend wird kurzzeitig das Gehäuse mit Sauerstoff geflutet und die darin befindliche Luft ausgetauscht. Der Patient atmet dann reinen Sauerstoff bzw. ein Sauerstoffgemisch ein, wobei gleichzeitig dessen ganze Haut umspült wird. Vorzugsweise wird in diesen Fällen die Bestrahlungsquelle innerhalb des Gehäuses angeordnet. Danach werden zum einen die Reflexions- und Absorptionsverluste am Hüllkörper vermieden und zum anderen kann die Bestrahlungsquelle näher am Patienten angeordnet werden, so daß sich höhere Bestrahlungsstärken einstellen. Unter Umständen kann es vorteilhaft sein, der gasdichten Bestrahlungskammer hyperbaren Sauerstoff zuzuführen, um bei besonders ausgeprägten Fällen eine ausreichende Oxygenierung zu erreichen.

In einer weiteren bevorzugten Ausführungsform umfaßt die Bestrahlungsanordnung eine Kühleinrichtung für die zu behandelnde Fläche. Dabei wird vorzugsweise mittels der Kühleinrichtung die zu bestrahlende Fläche unter die Normaltemperatur gekühlt. Sehr gute Ergebnisse wurden dabei mit lokalen Temperaturen im Bereich von <30°C erreicht. Vorzugsweise wird die Temperatur auf 10-20°C abgesenkt. Durch die Abkühlung kommt es zu einer Verringerung der Stoffwechselaktivität, wodurch der Sauerstoffverbrauch reduziert wird, so daß eine erhöhte Sauerstoffkonzentration in den zu behandelnden Geweben aufgebaut werden kann.

Insbesondere bei Ganzkörperbestrahlungen wird die Kühleinrichtung durch eine Wasserlage gebildet, in der dann der Patient liegt, wobei die Wassertemperatur jeweils einige Grad unter der angestrebten Hauttemperatur liegt.

In einer weiteren bevorzugten Ausführungsform umfaßt die Bestrahlungsanordnung einen Sensor zur Erfassung des Hautwiderstandes, wobei die Kühlleistung der Kühleinrichtung derart nachführbar ist, daß der Hautwiderstand konstant gehalten wird. Dadurch soll sichergestellt werden, daß die Kühlwirkung ausreichend groß ist, um eine Transpiration des Patienten zu verhindern, da der Schweiß von Patienten von Autoimmun-Erkrankungen wieder zu autoallergischen Reaktionen führen kann. Daher wird auch die direkte Wasserkühlung, bei der die zu behandelnde Haut unter 30°C gehalten wird, gegenüber Luftkühlung bevorzugt, die die Verdunstungskälte des Schweißes ausnutzt.

In einer weiteren bevorzugten Ausführungsform ist die breitbandige optische Bestrahlungsquelle als Plasmaentladungslampe mit einem der nachfolgenden Leuchtstoffe BaEuMgAl₁₀O₁₇, (SrBaCa)₁₀ (PO₄)₆ Cl₂:Eu, Ba oder Mg-Aluminat : Eu ausgebildet.

Durch die Ausbildung einer Leuchtstofffolie aus Silikonelastomere, in das die Leuchtstoffpartikel eingebettet sind, können einerseits Folien ausreichender Dicke mit einer genügend hohen Leuchtstoffkonzentration hergestellt werden. Des weiteren sind die Leuchtstoffpartikel luftdicht und wasserfrei in dem Silikonelastomere vernetzt, so daß diese keinem Alterungsprozeß ausgesetzt sind. Silikonelastomere sind UVC- durchlässig und weisen gegenüber alternativen UVC- durchlässigen Trägermaterialien erhebliche Vorteile auf. Saphir und Quarz sind zwar UVC- durchlässig, jedoch ist es aus leuchtstoffchemischen Gründen nicht möglich, anorganische Leuchtstoffe als Dotierung in Quarzfenster einzusetzen. Eine Saphirdotierung scheidet wegen der extremen Schmelztemperaturen von vornherein aus. Die Silikonelastomere sind bis 250°C stabil und benötigen keine Weichmacher oder andere flüchtige Substanzen, die abdampfen könnten. Aufgrund der verlängerten Lebensdauer des Leuchtstoffes, dadurch daß Leuchtstoff außerhalb des Ladungsgefäßes angeordnet werden kann und somit keine Reaktion mit dem Elektrodenabbrand auftreten kann, erhöht sich die Lebensdauer und Qualität der Plasmaentladungslampe. Neben den beschriebenen Silikonelastomeren kommen prinzipiell auch lichtdurchlässige Kunststoffe als Leuchtstofffolie in Betracht, da diese im Vergleich zu den Silikonelastomeren erheblich billiger sind. In Frage kommen dabei insbesondere Acrylate, transparentes PVC, Polyethylen oder Teflon. Diese sind hinsichtlich Wärmestabilität und Ausdampfung reizender Stoffe zu optimieren.

Aufgrund der Tatsache, daß anorganische Leuchtstoffe empfindlich auf Wasser oder Alkohol reagieren, wird das Silikonelastomer vorzugsweise durch eine Additionsvernetzung gebildet, wo im Gegensatz zu den bekannten Kondensationsvernetzungen keine Nebenprodukte wie beispielsweise Wasser freigesetzt werden. Des weiteren sind die durch Additionsvernetzung gebildeten Silikonpolymere hydrophob, da sie mehr oder weniger aus apolaren Kohlenwasserstoff-Ketten bestehen. Hierdurch ist sichergestellt, daß diese vollständig wasser- und feuchtigkeitsfrei bleiben. Ein weiterer Vorteil ist ein im Regelfall höheres E-Modul und eine hohe Zerreißfestigkeit.

In einer bevorzugten Ausführungsform ist das Silikonelastomere durch ein Verfahren herstellbar, bei dem ein Hydroxylpolydiorganosiloxan mit einem Organohydrogensiloxan unter Zuführung der Leuchtstoffpartikel in kristalliner Form vorliegen können. Mittels eines Platinkatalysators ist dann bei Raumtemperatur eine chemische Reaktion erzeugbar, die zu einer vollständigen Vernetzung führt, wobei aufgrund der geringen Prozeßtemperaturen die Leuchtstoffpartikel nicht belastet werden.

Die Dicke der Leuchtstofffolie liegt vorzugsweise im Bereich zwischen 10- 800 µm, wobei die Flächendichte der Leuchtstoffe dabei zwischen 1 - 100 mg/cm² beträgt. Besonders vorteilhaft erscheinen Dicken zwischen 100- 600 µm mit einer Flächendichte zwischen 5- 50 mg/cm². Die Korngröße der Leuchtstoffpartikel liegt dabei vorzugsweise zwischen 5-15 µm.

Durch die Anordnung der Leuchtstofffolie außerhalb des Entladungsraumes kann eine sehr flexibel handhabbare Bestrahlungsanordnung aufgebaut werden. Zum einem ist die Lebensdauer der Bestrahlungsanordnung nur noch durch die Plasmaentladungslampe selbst, insbesondere von deren Elektroden abhängig, da die Leuchtstofffolien selbst jederzeit einfach austauschbar sind. Dies ermöglicht darüber hinaus eine sehr einfache Bestückung mit verschieden dotierten Leuchtstofffolien, so daß sich mit einer Bestrahlungsanordnung unterschiedliche Spektralbereiche und Bestrahlungsstärken einstellen lassen.

In einer bevorzugten Ausführungsform ist in dem Entladungsrohr ein Verdrängungskörper angeordnet, so daß sich zwischen Entladungsrohr und Verdrängungskörper Kanäle ausbilden, wodurch die Plasmaentladungslampe sehr lang ausgeführt werden kann, ohne daß sehr große Zündspannungen benötigt werden, da immer noch ein ausreichend großes Plasmavolumen verbleibt. Andererseits steigt die emittierte Lichtenergiedichte in den Kanälen zwischen dem Entladungsrohr und dem Verdrängungskörper an.

In einer alternativen Ausführungsform ist anstelle der Leuchtstofffolie ein Pump-Kreislauf mit einer Silikonaufschlämmung um die Quecksilberniederdruckentladungslampe angeordnet, wobei die Leuchtstoffe sich in der Silikonaufschlämmung befinden. Dabei umströmt die Silikonaufschlämmung vorzugsweise direkt das Entladungsrohr. Aufgrund der Umströmung kann die auftretende Verlustwärme besser abgeführt werden, so daß die Plasmaentladungslampe mit höheren Leistungen betreibbar ist, ohne daß es zu thermischen Zerstörungseffekten der Leuchtstoffe kommt. Als Silikonaufschlämmung kommen insbesondere Silikonöle in Frage, wie beispielsweise DC 360 Medical Fluid der Firma Dow Corning Corporation oder Baysilone-ÖI-M oder -MPH der Firma GE-Bayer-Silicones.

In einer weiteren bevorzugten Ausführungsform ist die optische Bestrahlungsquelle als Metallhalogenidlampe mit einem Zündgas und Quecksilber sowie mit einem Metallhalogenidaditiv InJ₃ ausgebildet. Dabei ist das Gewichtsverhältnis zwischen Quecksilber und InJ₃ vorzugsweise zwischen 20 - 80. Zur Unterdrückung der nicht gewünschten Strahlung zwischen 340 - 430 nm bzw. 340 - 440 nm können entsprechende UVA- und Blaufilter zur Anwendung kommen. Hierzu kann beispielsweise um das eigentliche Entladungsrohr ein Hüllrohr aus Glas, einem transparenten UVAundurchlässigem Kunststoff wie beispielsweise Acryl GS oder Polycarbonat angeordnet sein, wobei der Hüllkörper zusätzlich mit Filterschichten für den Bereich zwischen 400 - 430/440 nm ausgebildet ist. Neben der Filterfunktion dient das Hüllrohr zur Wärmeisolation, da im Laufe des Betriebes die Oberfläche des Entladungsrohres sehr heiß werden kann. Des weiteren kommt es zur Entkopplung von äußeren Temperaturveränderungen, die sich negativ auf das Entladungsverhalten auswirken könnten. Bei Verwendung der zuvor beschriebenen Leuchtstoffe wird darüber hinaus ein Großteil der nicht gewünschten Strahlung mit Wellenlängen kleiner 430 nm in das gewünschte Wellenlängenintervall transformiert, so daß sich der Wirkungsgrad der Plasmaentladungslampe erhöht.

Zur Verbesserung des Wirkungsgrades wird das vorzugsweise als Quarzrohr ausgebildete Entladungsrohr der Metallhalogenidlampe im Bereich der Elektroden mittels Zirkonium teilverspiegelt, um die Temperatur im elektrodennahen Raum des Quarzkolbens zu erhöhen.

## Patentansprüche

1. Therapeutische Bestrahlungsanordnung, insbesondere zur chronischen Behandlung von ganz oder teilweise zellvermittelten Entzündungen der Haut, inneren Organe und viralen und infektiösen Erkrankungen , umfassend mindestens eine breitbandige optische Bestrahlungsquelle,
**dadurch gekennzeichnet, daß**
die Bestrahlungsquelle im Wellenlängenbereich von 430-500 nm eine Bestrahlungsstärke von mindestens 60 mW/cm² und im Wellenlängenbereich von 340-430 nm eine Bestrahlungsstärke von weniger als 20 mW/cm² erzeugt.

2. Therapeutische Bestrahlungsanordnung nach Anspruch 1, **dadurch gekennzeichnet, daß** die Bestrahlungsanordnung im Wellenlängenbereich von 440-480 nm eine Bestrahlungsstärke von mindestens 60 mW/cm² und im Wellenlängenbereich von 340-440 nm eine Bestrahlungsstärke von weniger als 20 mW/cm² erzeugt.

3. Therapeutische Bestrahlungsanordnung nach Anspruch 1 oder 2, **dadurch gekennzeichnet, daß** die Bestrahlungsstärke im Wellenlängenbereich von 430-500 bzw. 440-480 nm mindestens 100 mW/cm² ist.

4. Therapeutische Bestrahlungsanordnung nach einem der vorangegangenen Ansprüche, **dadurch gekennzeichnet, daß** die Bestrahlungsanordnung eine Einrichtung zur inspiratorischen und/oder topischen Sauerstoffzugabe umfaßt.

5. Therapeutische Bestrahlungsanordnung nach Anspruch 4, **dadurch gekennzeichnet, daß** die Einrichtung einen gasdichten Hüllkörper umfaßt, in dem eine reine Sauerstoffatmosphäre erzeugbar ist.

6. Therapeutische Bestrahlungsanordnung nach Anspruch 5, **dadurch gekennzeichnet, daß** die optische Bestrahlungsquelle im Hüllkörper angeordnet ist.

7. Therapeutische Bestrahlungsanordnung nach einem der vorangegangenen Ansprüche, **dadurch gekennzeichnet, daß** die Bestrahlungsanordnung eine Kühleinrichtung für eine zu bestrahlende Fläche umfaßt.

8. Therapeutische Bestrahlungsanordnung nach Anspruch 7, **dadurch gekennzeichnet, daß** die Kühleinrichtung einen Sensor zur Erfassung des Hautwiderstandes umfaßt und eine Kühlleistung der Kühleinrichtung zur Einhaltung eines konstanten Hautwiderstandes nachführbar ist.

9. Therapeutische Bestrahlungsanordnung nach Anspruch 7 oder 8, **dadurch gekennzeichnet, daß** mittels der Kühleinrichtung die zu behandelnde Fläche auf <30°C temperierbar ist.

10. Therapeutische Bestrahlungsanordnung nach einem der Ansprüche 7 bis 9, **dadurch gekennzeichnet, daß** die Kühleinrichtung als Wasserbad ausgebildet ist.

11. Therapeutischen Bestrahlungsanordnung nach einem der vorangegangenen Ansprüche, **dadurch gekennzeichnet, daß** die breitbandige optische Bestrahlungsquelle als Plasmaentladungslampe mit einem der nachfolgenden Leuchtstoffe Ba Eu Mg Al₁₀O₁₇, (SrBaCa)₁₀ (PO₄)₆ Cl₂:Eu, Ba oder Mg-Aluminat: Eu ausgebildet ist.

12. Therapeutische Bestrahlungsanordnung nach Anspruch 11, **dadurch gekennzeichnet, daß** die Leuchtstoffe in einer Leuchtstoffolie aus Silikonelastomere eingebettet sind.

13. Therapeutische Bestrahlungsanordnung nach Anspruch 12, **dadurch gekennzeichnet, daß** das Silikonelastomere durch folgende Verfahren herstellbar ist:
a) Mischen eines Hydroxylpolydiorganosiloxans mit einem Organohydrogensiloxan,
b) Zuführen von Leuchtstoffpartikeln und
c) Erzeugen einer chemischen Reaktion mittels eines Platinkatalysators bei Raumtemperatur.

14. Therapeutische Bestrahlungsanordnung nach einem der Ansprüche 12 oder 13 **dadurch gekennzeichnet, daß** die Leuchtstoffolie zwischen 10 bis 800 µm dick ist.

15. Therapeutische Bestrahlungsanordnung nach einem der Ansprüche 12 bis 14, **dadurch gekennzeichnet, daß** die Flächendichte der Leuchtstoffolie 1 - 100 mg/cm² beträgt.

16. Therapeutische Bestrahlungsanordnung nach einem der Ansprüche 12 bis 15, **dadurch gekennzeichnet, daß** die Korngröße der Leuchtstoffpartikel zwischen 5-15 µm beträgt.

17. Therapeutischen Bestrahlungsanordnung nach einem der Ansprüche 12 bis 16, **dadurch gekennzeichnet, daß** die Leuchtstoffolie auf der Außenseite eines Hüllkörpers angeordnet ist.

18. Therapeutische Bestrahlungsanordnung nach einem der Ansprüche 1 bis 11 **dadurch gekennzeichnet, daß** um die Plasmaentladungslampe ein Pump-Kreislauf mit einer Silikonaufschlämmung angeordnet ist, wobei die Leuchtstoffe sich in der Silikonaufschlämmung befinden.

19. Therapeutische Bestrahlungsanordnung nach einem der Ansprüche 1 bis 18, **dadurch gekennzeichnet, daß** die optische Bestrahlungsquelle als Metallhalogenidlampe mit einem Zündgas und Quecksilber sowie mit einem Metallhalogenidadditiv InJ₃ ausgebildet ist.

20. Therapeutische Bestrahlungsanordnung nach Anspruch 19, **dadurch gekennzeichnet, daß** das Gewichtsverhältnis zwischen Quecksilber und InJ₃ 20-80 beträgt.

21. Therapeutischen Bestrahlungsanordnung nach einem der Ansprüche 19 oder 20, **dadurch gekennzeichnet, daß** das Entladungsrohr der optischen Bestrahlungsquelle im Bereich der Elektroden mittels Zirkoniumoxid teilverspiegelt ist.

## Claims

1. Therapeutic irradiation arrangement, especially for the chronic treatment of inflammations of the skin, which are completely or partially cell-mediated, of inner organs and of viral and infectious diseases, said arrangement comprising at least one broadband optical radiation source, **characterised in that** the radiation source generates an irradiation intensity of at least 60 mW/cm² in the 430-500 nm wavelength range and an irradiation intensity of less than 20 mW/cm² in the 340-430 nm wavelength range.

2. Therapeutic irradiation arrangement according to claim 1, **characterised in that** the irradiation arrangement generates an irradiation intensity of at least 60 mW/cm² in the 440-480 nm wavelength range and an irradiation intensity of less than 20 mW/cm² in the 340-440 nm wavelength range.

3. Therapeutic irradiation arrangement according to claim 1 or 2, **characterised in that** the irradiation intensity is at least 100 mW/cm² in the 430-500 or 440-480 nm wavelength range.

4. Therapeutic irradiation arrangement according to one of the preceding claims, **characterised in that** the irradiation arrangement includes a device for the inspiratory and/or topical addition of oxygen.

5. Therapeutic irradiation arrangement according to claim 4, **characterised in that** the device includes a gas-tight enveloping body in which a pure oxygen atmosphere may be generated.

6. Therapeutic irradiation arrangement according to claim 5, **characterised in that** the optical radiation source is arranged in the enveloping body.

7. Therapeutic irradiation arrangement according to one of the preceding claims, **characterised in that** the irradiation arrangement includes a cooling device for a surface to be irradiated.

8. Therapeutic irradiation arrangement according to claim 7, **characterised in that** the cooling device includes a sensor for detecting the skin resistance and the cooling capacity of the cooling device may be tracked to maintain a constant skin resistance.

9. Therapeutic irradiation arrangement according to claim 7 or 8, **characterised in that** the temperature of the surface to be treated may be kept constant at <30°C by means of the cooling device.

10. Therapeutic irradiation arrangement according to one of claims 7 to 9, **characterised in that** the cooling device is in the form of a water bath.

11. Therapeutic irradiation arrangement according to one of the preceding claims, **characterised in that** the broadband optical radiation source is configured as a plasma discharge lamp with one of the following fluorescent materials Ba Eu Mg Al₁₀O₁₇, (SrBaCa)₁₀(PO₄)₆Cl₂:Eu, Ba or Mg-aluminate: Eu.

12. Therapeutic irradiation arrangement according to claim 11, **characterised in that** the fluorescent materials are embedded in a fluorescent film formed from silicone elastomer.

13. Therapeutic irradiation arrangement according to claim 12, **characterised in that** the silicone elastomer may be produced by the following methods:
a) mixing a hydroxyl polydiorganosiloxane with an organohydrogen siloxane,
b) supplying particles of fluorescent materials and
c) generating a chemical reaction by means of a platinum catalyst at room temperature.

14. Therapeutic irradiation arrangement according to one of claims 12 or 13, **characterised in that** the fluorescent film is between 10 and 800 µm thick.

15. Therapeutic irradiation arrangement according to one of claims 12 to 14, **characterised in that** the surface density of the fluorescent film is 1-100 mg/cm².

16. Therapeutic irradiation arrangement according to one of claims 12 to 15, **characterised in that** the particle size of the fluorescent material particles is between 5 and 15 µm.

17. Therapeutic irradiation arrangement according to one of claims 12 to 16, **characterised in that** the fluorescent film is arranged on the outside of an enveloping body.

18. Therapeutic irradiation arrangement according to one of claims 1 to 11, **characterised in that** a pump circuit with a silicone suspension is arranged around the plasma discharge lamp, the fluorescent materials being located in the silicone suspension.

19. Therapeutic irradiation arrangement according to one of claims 1 to 18, **characterised in that** the optical radiation source is in the form of a metal halide lamp with an ignition gas and mercury and with a metal halide additive InJ₃.

20. Therapeutic irradiation arrangement according to claim 19, **characterised in that** the weight ratio of mercury to InJ₃ is 20-80.

21. Therapeutic irradiation arrangement according to one of claims 19 or 20, **characterised in that** the discharge tube of the optical radiation source is partly mirror-coated by means of zirconium oxide in the region of the electrodes.

## Revendications

1. Agencement d'irradiation thérapeutique, en particulier pour le traitement chronique d'inflammations de la peau à médiation cellulaire complète ou partielle, des organes internes et des affections virales et infectieuses, comprenant au moins une source d'irradiation optique à large bande,
**caractérisé en ce que**
la source d'irradiation dans la plage de longueur d'onde de 430 à 500 nm génère une intensité d'irradiation d'au moins 60 mW/cm² et dans la plage de longueur d'onde de 340 à 430 nm une intensité d'irradiation inférieure à 20 mW/cm².

2. Agencement d'irradiation thérapeutique selon la revendication 1, **caractérisé en ce que** l'agencement d'irradiation génère dans la plage de longueur d'onde de 440 à 480 nm une intensité d'irradiation d'au moins 60 mW/cm² et dans la plage de longueur d'onde de 340 à 440 nm une intensité d'irradiation inférieure à 20 mW/cm².

3. Agencement d'irradiation thérapeutique selon la revendication 1 ou 2, **caractérisé en ce que** l'intensité d'irradiation dans la plage de longueur d'onde de 430 à 500 et/ou de 440 à 480 nm est au moins de 100 mW/cm².

4. Agencement d'irradiation thérapeutique selon l'une des revendications précédentes, **caractérisé en ce que** l'agencement d'irradiation comporte un dispositif destiné à l'ajout d'oxygène inspiratoire et/ou topique.

5. Agencement d'irradiation thérapeutique selon la revendication 4, **caractérisé en ce que** le dispositif comporte un corps creux étanche au gaz, dans lequel peut être générée une atmosphère d'oxygène pure.

6. Agencement d'irradiation thérapeutique selon la revendication 5, **caractérisé en ce que** la source d'irradiation optique est disposée dans le corps creux.

7. Agencement d'irradiation thérapeutique selon l'une des revendications précédentes, **caractérisé en ce que** l'agencement d'irradiation comporte un dispositif de refroidissement pour une surface destinée à être irradiée.

8. Agencement d'irradiation thérapeutique selon la revendication 7, **caractérisé en ce que** le dispositif de refroidissement comporte un capteur destiné à détecter la résistance de la peau et **en ce qu'**une puissance frigorifique du dispositif de refroidissement peut être réglée pour respecter une résistance de la peau constante.

9. Agencement d'irradiation thérapeutique selon la revendication 7 ou 8, **caractérisé en ce que** la surface destinée à être traitée peut être tempérée à <30°C au moyen du dispositif de refroidissement.

10. Agencement d'irradiation thérapeutique selon l'une des revendications 7 à 9, **caractérisé en ce que** le dispositif de refroidissement est conçu comme un bain-marie.

11. Agencement d'irradiation thérapeutique selon l'une des revendications précédentes, **caractérisé en ce que** la source d'irradiation optique à large bande est conçue comme une lampe plasma à décharge comprenant une des substances fluorescentes suivantes Ba Eu Mg Al₁₀O₁₇, (SrBaCa)₁₀(PO₄)₆Cl₂:Eu, Ba ou Mg-aluminate:Eu.

12. Agencement d'irradiation thérapeutique selon la revendication 11, **caractérisé en ce que** les substances fluorescentes sont encastrées dans un film fluorescent en élastomères à la silicone.

13. Agencement d'irradiation thérapeutique selon la revendication 12, **caractérisé en ce que** les élastomères à la silicone peuvent être fabriqués par les procédés suivants :
a) mélange d'un hydroxylpolydiorganosiloxane avec un organo hydrogènosiloxane,
b) amenée de particules fluorescentes et
c) génération d'une réaction chimique au moyen d'un catalyseur de platine à température ambiante.

14. Agencement d'irradiation thérapeutique selon l'une des revendications 12 ou 13, **caractérisé en ce que** l'épaisseur du film fluorescent est comprise entre 10 et 800 µm.

15. Agencement d'irradiation thérapeutique selon l'une des revendications 12 à 14, **caractérisé en ce que** la densité superficielle du film fluorescent est de 1 à 100 mg/cm².

16. Agencement d'irradiation thérapeutique selon l'une des revendications 12 à 15, **caractérisé en ce que** la taille de grain des particules fluorescentes est comprise entre 5 et 15 µm.

17. Agencement d'irradiation thérapeutique selon l'une des revendications 12 à 16, **caractérisé en ce que** le film fluorescent est disposé sur le côté extérieur d'un corps creux.

18. Agencement d'irradiation thérapeutique selon l'une des revendications 1 à 11, **caractérisé en ce qu'**un circuit de pompage comprenant une suspension de silicone est disposé autour de la lampe plasma à décharge, les substances fluorescentes se trouvant dans la suspension de silicone.

19. Agencement d'irradiation thérapeutique selon l'une des revendications 1 à 18, **caractérisé en ce que** la source d'irradiation optique est conçue comme une lampe aux halogénures métalliques comprenant un gaz inflammable et du mercure ainsi qu'un additif d'halogénures métalliques InJ₃.

20. Agencement d'irradiation thérapeutique selon la revendication 19, **caractérisé en ce que** le rapport de poids entre le mercure et le INJ₃ est de 20 à 80.

21. Agencement d'irradiation thérapeutique selon l'une des revendications 19 ou 20, **caractérisé en ce que** le tuyau de décharge de la source d'irradiation optique est en partie métallisé par vaporisation d'aluminium dans la zone des électrodes au moyen de dioxyde de zirconium.
